(19) Europäisches Patentamt
European Patent Office
Office européen des brevets

(11) Veröffentlichungsnummer: **0 010 656**
**B1**

(12) # EUROPÄISCHE PATENTSCHRIFT

(45) Veröffentlichungstag der Patentschrift: **22.04.81**

(51) Int. Cl.³: **C 07 C 69/145,** C 07 C 69/07, C 07 C 69/24

(21) Anmeldenummer: **79103888.8**

(22) Anmeldetag: **10.10.79**

(54) **Verfahren zur Herstellung von 4-Acyloxy-2-methyl-crotonaldehyden.**

(30) Priorität: **16.10.78 DE 2844949**

(43) Veröffentlichungstag der Anmeldung: **14.05.80 Patentblatt 80/10**

(45) Bekanntmachung des Hinweises auf die Patenterteilung: **22.04.81 Patentblatt 81/16**

(84) Benannte Vertragsstaaten: **CH DE FR GB NL**

(56) Entgegenhaltungen:
**DE-A1-2 621 224**

**JOURNAL OF THE AMERICAN CHEMICAL SOCIETY, Band 54, November 1932 A.F. SHEPARD et al. "Rearrangement of unsaturated 1.4-glycols. 2-Methyl-2-butene-1.4-diol" Seiten 4385 bis 4391**

**CHEMISCHE BERICHTE, Band 90, Nr. 2, 1957, Weinheim H. H. INHOFFEN et al. «Über die stereoisomeren 1.4-Diacetoxy-butadiene-(1.3)» Seiten 187 bis 193**

(73) Patentinhaber: **BASF Aktiengesellschaft, Carl-Bosch-Strasse 38, D-6700 Ludwigshafen (DE)**

(72) Erfinder: **Fischer, Rolf, Dr. Dipl.-Chem., Bergstrasse 98, D-6900 Heidelberg (DE)**
Erfinder: **Weitz, Hans-Martin, Dr. Dipl.-Chem., Auf dem Koeppel 40, D-6702 Bad Duerkheim 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).

ACTORUM AG.

EP 0 010 656 B1

# Verfahren zur Herstellung von 4-Acyloxy-2-methylcrotonaldehyden

Die Erfindung betrifft ein Verfahren zur Herstellung von 4-Acyloxy-2-methylcrotonaldehyden durch Umsetzung von 1,1,4-Triacyloxy-2-methyl-2-butenen mit Wasser in Gegenwart von Carbonsäuren.

Es ist bekannt, dass man Gemische aus (E)- und (Z)-4-Acetoxy-crotonaldehyd erhält, wenn man 1,1,4-Triacetoxy-2-buten mit Wasser zum Rückfluss erhitzt, das Reaktionsgemisch mit Äther extrahiert und die vereinigten Ätherextrakte fraktioniert destilliert. Die Ausbeute an 4-Acetoxy-crotonaldehyden beträgt nur 40%. (Chemische Berichte, Band 90, Seiten 187–193, 1957).

Es bestand die Aufgabe, die für die Synthese von Derivaten des all-trans Vitamins A als Vorprodukte geeigneten 4-Acyloxy-2-methylcrotonaldehyde aus 1,1,4-Triacyloxy-2-methyl-2-butenen herzustellen. Dabei war es wünschenswert, aus Isomerengemischen von (E)- und (Z)-1,1,4-Triacyloxy-2-methyl-2-butenen in einem Reaktionsschritt und ohne zusätzliche Isomerisierungs- oder Trennstufe die (E)-Isomeren der 4-Acyloxy-2-methylcrotonaldehyde zu erhalten, weil nur diese für die Herstellung der all-trans Vitamin-A-Derivate geeignet sind.

Es wurde gefunden, dass man 4-Acyloxy-2-methylcrotonaldehyde (4-Acyloxy-2-methyl-2-buten-1-ale) der Formel

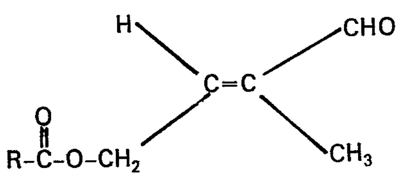

I,

worin R ein Wasserstoffatom oder einen aliphatischen Rest mit 1 bis 5 Kohlenstoffatomen bedeutet, in hoher Ausbeute und fast ausschliesslich als gewünschtes trans-Isomeres erhält, wenn man 1,1,4-Triacyloxy-2-methyl-2-butene der Formel

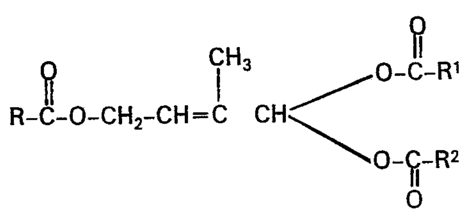

II,

in der $R^1$ und $R^2$ jeweils ein Wasserstoffatom oder einen aliphatischen Rest mit 1 bis 5 Kohlenstoffatomen bedeuten, mit Wasser in Gegenwart von Carbonsäuren der Formel

R-COOH III,

wobei R die oben genannte Bedeutung besitzt, umsetzt.

Die Reaktion kann für den Fall der Herstellung von 4-Acetoxy-2-methyl-crotonaldehyd (4-Acetoxy-tiglinaldehyd) durch die folgenden Formeln wiedergegeben werden ($-OAc= -O--CO-CH_3$):

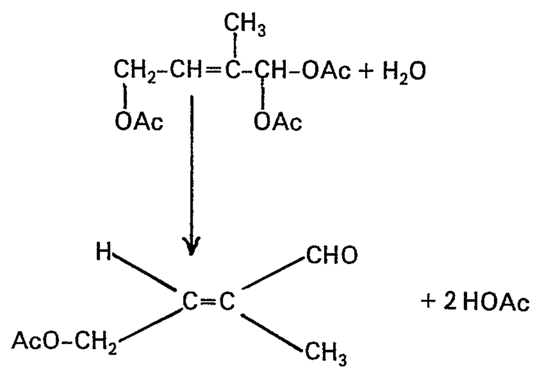

Das Ergebnis der erfindungsgemässen Verseifung, bei der auch aus Gemischen von (E)- und (Z)-1,1,4-Triacyloxy-2-methyl-2-butenen die gewünschten (E)-4-Acyloxy-2-methylcrotonaldehyde entstehen, ist überraschend, da sich bekanntlich spezifische cis-trans-Isomerisierungen in der Mehrzahl der Fälle nur photochemisch, in Gegenwart von radikalischen Katalysatoren, wie Stickstoffmonoxid oder Jod oder in Einzelfällen thermisch herbeiführen lassen. (Houben-Weyl, Methoden der organischen Chemie, Band 5/1b, Seiten 670 bis 672.) Bei der Hydrolyse von Gemischen der (E)- und (Z)-Isomeren der Formel II wären Gemische aus (E)- und (Z)-4-Acyloxy-2-methylcrotonaldehyden und ausserdem wesentlich schlechtere Ausbeuten zu erwarten gewesen.

Die als Ausgangsstoffe verwendeten 1,1,4-Triacyloxy-2-methyl-2-butene können z.B. durch Acyloxylierung von 1-Acyloxy-2-methyl-1,3-butadienen mit Carbonsäuren und Sauerstoff in Gegenwart von Palladium und Platin enthaltenden Katalysatoren hergestellt werden. Das Verhältnis von (E)- zu (Z)-Isomeren ist für die erfindungsgemässe Verseifung nicht von Bedeutung, da man, ausgehend von den reinen Isomeren und von Gemischen beliebiger Zusammensetzung, fast ausschliesslich die gewünschten (E)-4-Acyloxy-2-methylcrotonaldehyde erhält.

Die Reste R, $R^1$ und $R^2$, die gleich oder verschieden sein können, stehen vorzugsweise für ein Wasserstoffatom oder für einen Alkylrest mit 1 bis 5 C-Atomen, insbesondere für einen Methyl-, Äthyl-, Propyl-, n-Butyl oder i-Butyl-Rest.

Die Umsetzung wird in Gegenwart von Carbonsäuren der Formel III, in denen R die obengenannte Bedeutung hat, durchgeführt. Essigsäure ist aus wirtschaftlichen und praktischen Gründen besonders bevorzugt. Zweckmässigerweise wird die Umsetzung in Gegenwart der Carbonsäure durchgeführt, die auch bei der Herstellung der 1,1,4-Triacyloxy-2-methyl-2-butene als Reaktionspartner und/oder Reaktionsmedium verwendet wird. Man verwendet z.B. 0,1 bis 50 Mol Carbonsäure der Formel III pro Mol Triacylat der Formel II.

Die Umsetzung wird im allgemeinen bei Temperaturen von 0 bis 200 °C, vorzugsweise von 80 bis 200 °C, drucklos oder unter Druck, diskontinuierlich oder kontinuierlich durchgeführt. Die Wassermenge beträgt vorteilhaft 1 bis 50 Mol, insbesondere 1 bis 5 Mol Wasser je Mol Ausgangsstoff der Formel II.

Beispielsweise verfährt man wie folgt: ein Gemisch aus dem Ausgangsstoff der Formel II, Wasser und der Carbonsäure der Formel III wird auf die Reaktionstemperatur gebracht und 1 bis 5 Stunden bei dieser Temperatur gehalten. Dann wird nach der destillativen Abtrennung von Carbonsäuren und überschüssigem Wasser fraktioniert destilliert, um den (E)-4-Acyloxy-2-methylcrotonaldehyd zu gewinnen.

Die nach dem Verfahren der Erfindung erhältlichen 4-Acyloxy-2-methylcrotonaldehyde stellen wertvolle Ausgangsstoffe für die Herstellung von Terpenen dar. (E)-4-Acetoxy-2-methylcrotonaldehyd dient beispielsweise bei der Synthese von Vitamin A-Acetat als $C_5$-Baustein.

Die in den Beispielen genannten Teile sind Gewichtsteile.

Beispiel 1

48,8 Teile 1,1,4-Triacetoxy-2-methyl-2-buten, die nach der NMR-Analyse aus 46,4% (E)- und 53,6% (Z)-Isomeren bestehen, werden mit 500 Teilen Eisessig und 18 Teilen Wasser 2,5 Stunden lang zum Sieden erhitzt. Nach dem Abziehen von Essigsäure und Wasser am Rotationsverdampfer bei 40 bis 50 °C und Wasserstrahlvakuum erhält man bei der fraktionierten Destillation 23,2 Teile 4-Acetoxy-2-methylcrotonaldehyd (81,6% der Theorie) vom Siedepunkt 102 bis 103 °C/23,9 mbar ($n_D^{20}$ = 1,4641). Nach der NMR-Analyse (Aldehydprotonen) besteht der gewonnene Aldehyd zu 98,0% aus (E)- und zu 2,0 % aus (Z)-4-Acetoxy-2-methylcrotonaldehyd.

Beispiel 2

48,8 Teile 1,1,4-Triacetoxy-2-methyl-2-buten der im Beispiel 1 angegebenen Isomerenzusammensetzung werden mit 400 Teilen Ameisensäure und 10,8 Teilen Wasser 3 Stunden lang zum Sieden erhitzt. Nach dem Abziehen von Ameisensäure und Wasser am Rotationsverdampfer erhält man bei der fraktionierten Destillation 21,5 Teile 4-Acetoxy-2-methylcrotonaldehyd (75,6% der Theorie) vom Siedepunkt 102 bis 105 °C/21 mbar ($n_D^{20}$ = 1,4642). Nach der NMR-Analyse besteht der gewonnene Aldehyd zu 98,3% aus (E)- und zu 1,7% aus (Z)-4-Acetoxy-2-methylcrotonaldehyd.

Beispiel 3

40,3 Teile 1,1,4-Triacetoxy-2-methyl-2-buten der in Beispiel 1 angegebenen Isomerenzusammensetzung werden mit 80 Teilen Wasser und 12 Teilen Eisessig 3 Stunden unter Rückfluss erhitzt.

Die Lösung wird nach dem Abkühlen mit 50 Volumenteilen Äther extrahiert, mit Calciumchlorid gesättigt und noch dreimal mit je 50 Volumenteilen Äther extrahiert. Die vereinigten Ätherextrakte werden mit Magnesiumsulfat getrocknet. Nach dem Abziehen des Äthers werden bei fraktionierter Destillation 18,8 Teile 4-Acetoxy-2-methylcrotonaldehyd (80,1% der Theorie) vom Siedepunkt 100 bis 104 °C/18,6 mbar ($n_D^{20}$ = 1,4642) erhalten. Nicht umgesetztes 1,1,4-Triacetoxy-2-methyl-2-buten ist nicht feststellbar. Laut NMR-Analyse besteht der gewonnene Aldehyd zu 97,5% aus (E)- und zu 2,5% aus (Z)-4-Acetoxy-2-methylcrotonaldehyd.

Beispiel 4

48,8 Teile 1,1,4-Triacetoxy-2-methyl-2-buten, die nach NMR-Analyse aus 75,5% (E)- und 24,5 (Z)-Isomeren bestehen, werden mit 250 Teilen Eisessig und 18 Teilen Wasser 3 Stunden lang zum Sieden erhitzt. Nach der in Beispiel 1 beschriebenen Aufarbeitung erhält man 23,9 Teile 4-Acetoxy-2-methylcrotonaldehyd (84,1% der Theorie) vom Siedepunkt 92 bis 96 °C/16 mbar ($n_D^{20}$ = 1,4638). Nach NMR-Analyse besteht der gewonnene Aldehyd zu 98,5% aus (E)- und zu 1,5% aus (Z)-4-Acetoxy-2-methylcrotonaldehyd.

Vergleichsbeispiel

Entsprechend dem in Chemische Berichte, Band 90 (1957), Seite 192, Versuch 1 beschriebenen Verfahren werden 40,3 Teile 1,1,4-Triacetoxy-2-methyl-2-buten, die nach der NMR-Analyse aus 46,4% (E)- und 53,6% (Z)-Isomeren bestehen, mit 80 Teilen Wasser 40 Minuten lang zum Sieden erhitzt. Die gaschromatographische Analyse einer Probe aus der auch bei Siedetemperatur sich abtrennenden organischen Phase ergibt, dass nur rund 10% des Ausgangsproduktes in 4-Acetoxy-2-methylcrotonaldehyd übergegangen sind. Man erhitzt das Reaktionsgemisch weitere 140 Minuten zum Sieden. Die nun homogene Lösung wird nach dem Abkühlen mit 50 Volumenteilen Äther extrahiert, mit Calciumchlorid gesättigt und noch dreimal mit je 50 Volumenteilen Äther extrahiert. Die vereinigten Ätherextrakte trocknet man mit Magnesiumsulfat. Sie ergeben nach Abziehen des Äthers bei der fraktionierten Destillation 12,6 Teile 4-Acetoxy-2-methylcrotonaldehyd (53,8% der Theorie) vom Siedepunkt 103 bis 106 °C/20 mbar ($n_D^{20}$ = 1,4640) und 9,1 Teile unumgesetztes 1,1,4-Triacetoxy-2-methyl-2-buten (22,6% der Theorie) vom Siedepunkt 118 bis 127 °C/20 mbar ($n_D^{20}$ = 1,4487). Nach der NMR-Analyse besteht der gewonnene Aldehyd zu 89,3% aus (E)- und zu 10,7% aus (Z)-4-Acetoxy-2-methylcrotonaldehyd.

**Patentansprüche**

1. Verfahren zur Herstellung von 4-Acyloxy-2-methylcrotonaldehyden der Formel

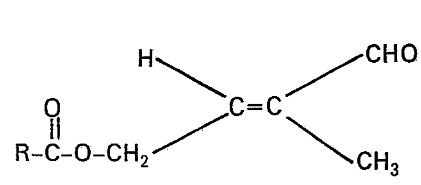

I,

worin R ein Wasserstoffatom oder einen aliphatischen Rest mit 1 bis 5 Kohlenstoffatomen bedeutet, dadurch gekennzeichnet, dass man 1,1,4-Triacyloxy-2-methyl-2-butene der Formel

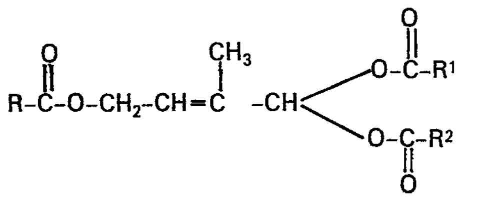

II,

in der $R^1$ und $R^2$ jeweils ein Wasserstoffatom oder einen aliphatischen Rest mit 1 bis 5 Kohlenstoffatomen bedeuten, mit Wasser in Gegenwart von Carbonsäuren der Formel

R-COOH III,

wobei R die oben genannte Bedeutung besitzt, umsetzt.

2. Verfahren nach Anspruch 1, dadurch gekennzeichnet, dass man als Verbindung der Formel II 1,1,4-Triacetoxy-2-methyl-2-buten verwendet.

3. Verfahren nach Anspruch 1, dadurch gekennzeichnet, dass man als Carbonsäuren der Formel III Essigsäure verwendet.

**Claims**

1. A process for the preparation of a 4-acyloxy-2-methyl-crotonaldehyde of the formula

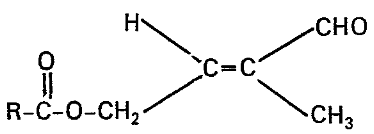

I

where R is hydrogen or an aliphatic radical of 1 to 5 carbon atoms, wherein a 1,1,4-triacyloxy-2-methyl-but-2-ene of the formula

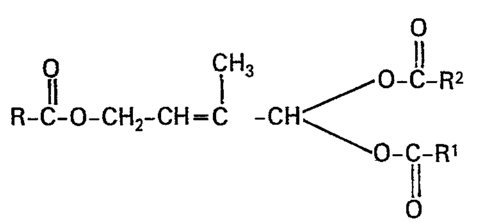

II

where $R^1$ and $R^2$ are each hydrogen or an aliphatic radical of 1 to 5 carbon atoms, is reacted with water in the presence of a carboxylic acid of the formula

R-COOH III

where R has the above meanings.

2. A process as claimed in claim 1, wherein the compound of the formula II is 1,1,4-tri acetoxy-2-methyl-but-2-ene.

3. A process as claimed in claim 1, wherein the carboxylic acid of the formula III is acetic acid.

**Revendications**

1. Procédé de préparation d'aldéhydes 4-acyloxy-2-méthyl-crotoniques répondant à la formule

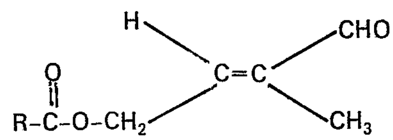

I,

dans laquelle R représente un atome d'hydrogène ou un reste aliphatique en C1-C5, caractérisé en ce que l'on fait réagir des 1,1,4-triacyloxy-2-méthyl-2-butènes répondant à la formule

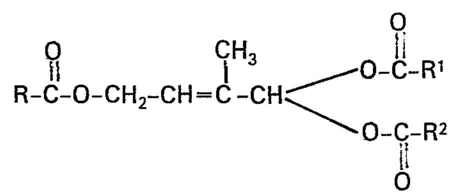

II,

dans laquelle $R^1$ et $R^2$ représentant chacun un atome d'hydrogène ou un reste aliphatique en C1-C5, avec de l'eau en présence d'acides carboxyliques répondant à la formule

R-COOH III,

dans laquelle R a les significations indiquées cidessus.

2. Procédé selon la revendication 1, caractérisé en ce que l'on utilise en tant que composé de formule II le 1,1,4-triacétoxy-2-méthyl-2-butène.

3. Procédé selon la revendication 1, caractérisé en ce que l'on utilise en tant qu'acide carboxylique de formule III l'acide acétique.